# EUROPÄISCHE PATENTSCHRIFT

(11) **EP 3 146 105 B1**
(45) Veröffentlichungstag und Bekanntmachung des Hinweises auf die Patenterteilung: **24.11.2021**
(21) Anmeldenummer: 15723523.5
(22) Anmeldetag: 20.05.2015
(51) Int. Cl.: D06H 1/04, D03D 15/56, A61F 13/00

(54) **ELASTISCHE BINDE**
ELASTIC BANDAGE
BANDE ÉLASTIQUE

(30) Priorität: 20.05.2014 DE 102014209591
(43) Veröffentlichungstag der Anmeldung: 29.03.2017
(73) Patentinhaber: Karl Otto Braun GmbH & Co. KG., 67752 Wolfstein (DE)
(72) Erfinder: SZOMBACH, Karlheinz, 67742 Lauterecken (DE); JUNG, Harald, 67757 Kreimbach-Kaulbach (DE); KLOEPPELS, Michael, 52249 Eschweiler (DE); GROSS, Helmut, 52223 Stolberg (DE); LANGEN, Guenter, 67752 Wolfstein (DE)
(74) Vertreter: DREISS Patentanwälte PartG mbB
(86) Internationale Anmeldenummer: PCT/EP2015/061165
(87) Internationale Veröffentlichungsnummer: WO 2015/177239

(56) Entgegenhaltungen:
- WO-A1-96/31175

## Beschreibung

Die Erfindung betrifft eine elastische Binde umfassend ein gewebtes Flächengebilde mit Schussfäden sowie elastischen Kettfäden, wodurch die Binde eine Elastizität in Längsrichtung erhält.

Eine solche Binde ist dabei insbesondere in Leinwandbindung gewebt, Grundsätzlich denkbar sind jedoch auch beliebige andere Bindungsarten, wobei auch zusätzliche weitere Fäden, wie Dreherfäden etc. vorgesehen sein können. Derartige Binden finden z. B. als sogenannte Kompressionsbinden Verwendung. Insbesondere werden solche Binden oder Bandagen als Kurzzugbinden, die einen hohen Arbeitsdruck bei geringem Ruhedruck gewährleisten und zugleich für den Patienten komfortabel zu tragen sind, eingesetzt. Darüber hinaus besteht ein Bedarf an Binden und Bandagen, die ein hohes Maß an Therapiesicherheit bieten.

Im Folgenden sollen die Begriffe "Bandage" und "Binde" synonym verwendet werden.

Bei der Verwendung einer derartigen Binde ist es wesentlich, dass der richtige vorgegebene Druck auf eine Gliedmaße eines Patienten aufgebracht wird. Es besteht dabei die Gefahr, dass durch zu starke Dehnung einer elastischen Binde der aufgebrachte Druck, insbesondere im Ruhezustand, zu groß ist und es so zu einem Abschnüren der Gliedmaße kommt. Hierdurch können schwerwiegende gesundheitliche Störungen ausgelöst werden. Darüber hinaus ist es in umgekehrter Weise auch möglich, dass die Binde aus Angst vor einem zu starken Dehnen und einer zu starken Kompressionskraft mit einer geringeren als der notwendigen Dehnung angelegt wird, wodurch eine nicht ausreichende Kompressionswirkung erzielt wird und damit der gewünschte Therapieeffekt nicht erreicht werden kann. Das Vermeiden dieser beiden Effekte wird unter Therapiesicherheit verstanden.

Hierzu ist im Stand der Technik, beispielsweise aus der DE 10 2005 033 720 A1 vorbekannt, bei einer in Kettrichtung längselastischen Binde, die ebenfalls als Kompressionsbinde eingesetzt werden kann, dass der niedrige Ruhedruck annähernd über die gesamte Dehnbarkeit der Binde bestehen bleibt. D. h. die Binde kann annähernd über ihre gesamte Dehnbarkeit mit geringstem Kraftaufwand gedehnt werden. Erst bei einer relativ hohen Anlegedehnung steigt der Ruhedruck an.

Die im Stand der Technik bekannte Binde weist dabei einen möglichst flachen Kurvenverlauf im Kraftdehnungsbereich im Bereich von mindestens 2/3 der Gesamtdehnung auf. Erst nach Erreichen des zweiten Drittels der Kurve (Dehnbarkeit) ist ein deutlicher Anstieg der Kraft erwünscht. Hierdurch soll eine hohe Therapiesicherheit zur Verfügung gestellt werden, da über einen weiten Bereich der Anlegedehnung ein nahezu konstanter Arbeitsdruck eingestellt werden kann.

Des Weiteren ist beispielsweise aus der DE 20 2004 011 946 U1 ein elastisches Schlauchband bekannt, bei dem in das Innere des Schlauches eine Einlage aufgenommen ist, die die maximale Dehnbarkeit des Bandes bzw. des Bandabschnittes begrenzt. Derartige Bänder können als Spanngummis zur Ladesicherung in Kraftfahrzeugen etc. eingesetzt werden.

Eine Binde gemäß dem Oberbegriff des Anspruchs 1 ist aus WO 96/31175 A1 bekannt.

Ausgehend von diesem Stand der Technik ist es die Aufgabe der Erfindung, eine Binde bzw. Bandage bereitzustellen, die dem Anwender signalisiert, wenn eine elastische Binde ausreichend weit gedehnt ist, um einen für die Therapie erforderlichen Kompressionsdruck auf eine Gliedmaße eines Patienten aufzubringen.

Die Erfindung löst diese Aufgabe durch eine elastische Binde gemäß dem Oberbegriff, bei der auf das gewebte Flächengebilde wenigstens ein Stoppfaden aufgebracht ist, durch den eine vorgegebene Dehnungsgrenze oder Dehnungsschwelle der Binde anzeigbar ist.

Durch den Stoppfaden, der bei einer vorgegebenen Dehnungsgrenze oder Dehnungsschwelle die weitere Dehnung der elastischen Binde begrenzt oder zumindest erschwert, wird für den Benutzer ein Signal gegeben, wann er zum einen die richtige, d. h. eine ausreichende Dehnung, der elastischen Binde erreicht hat, um eine ausreichende Kompressionskraft an einer Gliedmaße bereitzustellen, und auf der anderen Seite werden Fehler des Falschanlegens durch zu starke Dehnung der Binde minimiert oder sogar vermieden.

Durch diese zusätzliche Dehnungsgrenze bzw. Dehnungsschwelle wird unterhalb der bestehenden bindeneigenen Dehnungsgrenze einer jeden elastischen Binde, die durch die Dehnung bis zum Zerreißen der Binde gegeben ist, eine einfache Möglichkeit für den Benutzer bereitgestellt, auch bei geringen therapeutischen Vorkenntnissen Fehlbedienungen der Binde sowohl durch zu schwaches als auch durch zu starkes Dehnen der Binde zu vermeiden.

Dabei wird der mindestens eine Stoppfaden auf das gewebte Flächengebilde aufgebracht und nicht, wie bereits im Stand der Technik bekannt, in dieses im Webprozess integriert.

Hierdurch ist der Vorteil gegeben, dass die Herstellung derartiger Binden noch weiter vereinfacht werden kann und eine Vorfertigung ermöglicht wird. So können zunächst unabhängig von der später einzustellenden Dehnungsschwelle oder Dehnungsgrenze Binden hergestellt werden, die sämtlich identisch ausgestaltet sind. Erst nach dem Weben der textilen Flächengebilde, die dann zu Binden konfektioniert werden, können dann die Stoppfäden entsprechend der gewünschten Dehnungsschwelle oder Dehnungsgrenze auf die Binde aufgebracht werden. Hierdurch wird die Einstellung der Dehnungsschwelle oder Dehnungsgrenze stark vereinfacht, da die entsprechenden Binden oder Bandagen fertig konfektioniert auf die gewünschte Dehnungsschwelle oder Dehnungsgrenze gedehnt werden können, um dann mit einem oder mehreren entsprechenden Stoppfäden versehen zu werden, die dann die vorgegebene Dehnungsschwelle oder Dehnungsgrenze bei weiteren Verwendungen signalisieren. Der Webprozess als solcher wird hierdurch nicht verändert.

Grundsätzlich besitzen derartige gewebte Flächengebilde zum einen eine in weiten Bereichen frei einstellbare Dehnbarkeit sowie insbesondere eine Kompressionskraft in Ruhe eines Patienten bei angelegter Binde von kleiner 800 cN/cm, insbesondere kleiner 350 cN/cm, insbesondere kleiner 300 cN/cm und insbesondere zwischen 300 und 150 cN/cm Bindenbreite im angelegten Zustand. Auf diese Weise wird ein sehr geringer Ruhedruck erzielt, wobei der Ruhedruck der ständige Druck ist, den die Binde in Ruhe, also bei erschlaffter Muskulatur, auf das Gewebe und die Gefäße, d. h. auf die zu behandelnde Körperstelle, ausübt. Die Kraft, die aufgewandt wird, um die Binde zu dehnen, entspricht demnach der Kraft, die als Ruhedruck auf das Gewebe, d. h. auf die zu behandelnde Körperstelle, wirkt. Der Arbeitsdruck ist dagegen der Druck, den der sich kontrahierende, arbeitende Muskel durch Umfangszunahme dem Widerstand der Kompressionsbinde entgegensetzt.

Die erfindungsgemäßen Binden können sowohl Kurzzug- als auch Langzugbinden sein, wobei die Vorsehung einer Dehnungsschwelle oder eines Dehnungsstopps gerade für Langzugbinden besonders vorteilhaft erscheint.

Es kann dabei vorgesehen sein, dass die Kettfäden als baumwollelastische Fäden ausgebildet sind.

Durch die Verwendung von baumwollelastischen Fäden werden die Trageeigenschaften der Binde verbessert. Insbesondere können die Kompressionseigenschaften, insbesondere der im medizinischen Sinne niedrige Ruhedruck und der hohe Arbeitsdruck im Vergleich zu herkömmlichen Kompressionsbinden auf Basis elastischer Polymere, wie Gummi, Polyurethanelastomere, texturierte Polyamid- bzw. Polyestergarne, die einen hohen Ruhedruck mit einem geringen Arbeitsdruck erzeugen, deutlich verbessert werden. Gegenüber herkömmlichen Kurzzugbinden auf Basis von Baumwollfäden sind die Kraftdehnungseigenschaften verbessert in Richtung eines gesteigerten niedrigen Ruhedrucks, der annähernd über die gesamt Dehnbarkeit der Binde bestehen bleibt. D. h. die Binde kann annähernd über ihre gesamte Dehnbarkeit mit geringstem Kraftaufwand gedehnt werden. Die Dehnbarkeit kann dabei bei baumwollelastischen Kurzzugbinden 30% bis 110% bevorzugt 40% bis 100% und besonders bevorzugt 50% bis 90% betragen (gemessen nach DIN 61632). Bei Langzugbinden auf Basis elastischer Polymere kann die Dehnbarkeit 30% bis 250%, insbesondere 30% bis 200% und besonders bevorzugt 30% bis 150% betragen (gemessen nach DIN 61632).

Eine weitere Möglichkeit zur Erhöhung bzw. Reduzierung der Dehnbarkeit wird über die Einstellung der Schussdichte gegeben.

Besonders bevorzugt weist eine erfindungsgemäße Binde bei einer Dehnung von kleiner 50 %, insbesondere kleiner 60 %, und ganz besonders bevorzugt kleiner 65 % (bezogen auf die Gesamtdehnung der Binde nach DIN 61632) eine Kompressionskraft bei Ruhe des Patienten bei angelegter Binde von kleiner 800 cN/cm Bindenbreite , insbesondere kleiner 350 cN/cm und insbesondere zwischen 300 cN/cm bis 150 cN/cm Bindenbreite auf. Damit weist eine erfindungsgemäße Binde eine Rückstellkraft größer 800 cN/cm Bindenbreite, insbesondere größer 350 cN/cm Bindenbreite und insbesondere größer 300 cN/cm Bindenbreite erst im Bereich von 80 % bis 100 % der Gesamtdehnbarkeit in Längsrichtung auf.

Es kann dabei vorgesehen sein, dass die Schussfäden im Wesentlichen unelastisch ausgestaltet sind. D. h. bei einer erfindungsgemäßen Binde, die z. B. baumwollelastische Kettfäden oder Kettfäden aus elastischen Polymeren verwendet und im Wesentlichen unelastische Schussfäden aufweist, wird eine unielastische Binde erzeugt. Alternativ können jedoch auch elastische Schussfäden eingesetzt werden, beispielsweise ebenfalls baumwollelastische Schussfäden, so dass dann neben einer Längselastizität der Binde auch eine Elastizität in Querrichtung gegeben ist und eine bielastische Binde erzeugt wird.

Sofern zum Erreichen einer hohen Schiebesicherheit des Gewebes das textile Flächengebilde anstelle in Leinwandbindung in Dreherbindung gewebt wird, können die Dreherfäden als elastische oder unelastische Fäden ausgebildet sein.

Zur Herstellung von baumwollelastischen Fäden werden entweder Zwirn oder Spinnkreppfäden verwendet, die in S- und/oder Z-Drehungsrichtung mit Kreppdrehung von ca. 1100 bis 2400 Touren/m, insbesondere mit mehr als 2000 Touren/m in Mit- oder Gegenspinndrahtrichtung versehen sind. Das Bindengewebe wird später einem Krumpfprozess unterzogen, wodurch die Baumwollelastizität ausgelöst wird. Hierbei können alle herkömmlichen Maschinen, die einen Krumpfprozess anwenden, zum Einsatz gebracht werden, wie z. B. die Krumpfmaschine Shrinkomat SP (Firma M-Tech Maschinenbau Gesellschaft mbH, Viersen, Deutschland).

Die zur Anzeige der Dehnungsschwelle oder Dehnungsgrenze vorgesehenen Stoppfäden bzw. des mindestens einen Stoppfadens kann dadurch erfolgen, dass dieser mittels Aufnähen, Übernähen, Aufsticken, Verschweißen oder Verkleben mit dem textilen Flächengebilde verbunden ist. Hierzu können zusätzliche Fixierfäden vorgesehen sein. Aufnähen bezeichnet ein Verfahren, bei dem ein Faden auf ein fertiges Flächengebilde aufgelegt wird und dann mit einem weiteren Faden nähtechnisch fixiert wird. Beim Übernähen ist der Stoppfaden selber der Faden, der auf einem fertigen Flächengebilde vernäht wird. Aufsticken bezeichnet die Sticktechnik mit all ihren Varianten. Der Stoppfaden kann dabei auf einem textilen Flächengebilde aufliegen oder aus dessen Ebene aufragen (Flottierung). Je nach Länge und Auswahl des Stoppfadens, z.B. als elastischer oder unelastischer Stoppfaden liegt dieser auf dem Gebilde in "Schlangenlinien" auf.

Sofern ein Verschweißen vorgesehen ist, kann das gewebte Flächengebilde beispielsweise thermoplastische Fasern aufweisen, die ein Verschweißen über thermisches oder Ultraschallverschweißen der Stoppfäden mit dem Gewebe ermöglichen. Dies ist auch bei der Auswahl des Materials der Stoppfäden zu berücksichtigen. Alternativ ist auch ein Verkleben denkbar.

Bei den Stoppfäden handelt es sich entweder um unelastische Fäden, wobei hier natürliche Pflanzenfasern, Fasern tierischen Ursprungs oder Synthesefasern eingesetzt werden können. Insbesondere können diese Fäden aus Baumwolle, Zellwolle, Flachs, Wolle, Viskose, Seide und/oder Synthesefasern bestehen, wobei Baumwolle und Viskose bevorzugt sind. Als Synthesefasern können hierbei bevorzugt Fasern aus Polyamid, Polyester, Polypropylen oder Polyacryl eingesetzt werden; insbesondere Polyamide und Polyester. Die Binden, insbesondere im Fall von Langzugbinden, können bevorzugt aus Polyurethan oder Elasthan bestehen oder dieses umfassen.

Alternativ können auch elastische Stoppfäden eingesetzt werden, die eine geringere Elastizität als das gewebte Flächengebilde (Grundgewebe) der Binde aufweisen. Elastische Stoppfäden bieten den Vorteil, besonders einfach eine Dehnungsschwelle realisieren zu können, anstelle eines definierten Dehnungsstopps.

Die Stoppfäden können Einfachgarne sein, aber auch Zwirne und Umwindegarne.

Es können dabei ein oder mehrere Stoppfäden mit dem Flächengebilde verbunden werden, wobei durch die Platzierung sowie die Anzahl der Stoppfäden die Dehnungsgrenze bzw. die Dehnungsschwelle genau an einen für die jeweilige Anwendung benötigen Dehnungswert angepasst werden kann.

Das Anbringen der Stoppfäden kann dabei dergestalt erfolgen, dass die elastische Binde bis zu einem benötigen Dehnungswert gedehnt wird und die Stoppfäden dann bei diesem gewünschten Dehnungswert, der als Dehnungsgrenze oder Dehnungsschwelle angezeigt werden soll, unter Spannung mit dem gewebten Flächengebilde verbunden werden, so dass dann nach einem Relaxieren der Binde es zu einem Inwellenlegen des mindestens einen Stoppfadens kommt, so dass die Binde nach wie vor bis zur Dehnungsgrenze oder Dehnungsschwelle elastisch dehnbar ist, jedoch bei Erreichen der Dehnungsschwelle oder Dehnungsgrenze der mindestens eine Stoppfaden das Erreichen des gewünschten Dehnungswertes anzeigt.

Der mindestens eine Stoppfaden liegt dann geschlängelt, d. h. mit einer Fadenreserve, auf dem gewebten Flächengebilde auf und wird beispielsweise durch einen Fixierfaden oder Haftpunkte auf dem Flächengebilde fixiert. Bei Dehnung des Flächengebildes, also der elastischen Binde in Längsrichtung, strafft sich der Stoppfaden bis zu dem Punkt, an dem er fast fadengerade in Längsrichtung des Flächengebildes liegt. An diesem Punkt setzt die Signalwirkung des Stoppfadens hinsichtlich des Dehnungsstops bzw. der Dehnungsschwelle ein.

Wird dagegen ein elastischer Stoppfaden verwendet, ist nicht zwingend ein Inwellenlegen des Stoppfadens notwendig oder nur in einem geringeren Maße. Ebenfalls ist es auch möglich, einen elastischen Stoppfaden auf eine ungedehnte Binde aufzubringen oder nur auf eine nicht bis zur gewünschten Dehnungsschwelle oder -grenze gedehnte Binde.

Dabei kann vorzugsweise die Binde in Längsrichtung eine Dehnbarkeit von 20% bis 100% und insbesondere von 30% bis 90%, insbesondere von 30% bis 70% aufweisen, bei der der mindestens eine Stoppfaden das Erreichen der Dehnungsschwelle und/oder -grenze signalisiert. Die mit der entsprechenden Dehnung einhergehende Kraft kann insbesondere im Bereich von 10 cN/cm bis 50 cN/cm, insbesondere von 20 cN/cm bis 40 cN/cm eingestellt werden.

Der mindestens eine Stoppfaden kann dabei eine Dicke aufweisen, die größer, gleich oder feiner als die der Schuss- oder Kettfäden ist.

Erfindungsgemäß kann der mindestens eine Stoppfaden an mindestens zwei Punkten, vorzugsweise jedoch an mindestens 1 bis 50 Punkten pro 10 cm Bindenlänge und mindestens 3 und 20 Punkten pro 10 cm Bindenlänge und vorzugsweise mindestens 5 bis 15 Punkten pro 10 cm Bindenlänge mit dem Flächengebilde der Binde verbunden sein.

Darüber hinaus kann auch zur noch leichteren auch visuellen Erkennbarkeit vorgesehen sein, dass sich der Stoppfaden deutlich visuell vom Flächengebilde abhebt und gegebenenfalls auch farblich hier gegenüber abgesetzt sein kann.

Dabei soll unter einem Dehnungsstop die tatsächliche Begrenzung der weiteren Dehnung der elastischen Binde verstanden werden, wobei bei einem Überschreiten des signalisierten Dehnungsstops es zu einem Zerreißen des mindestens einen Stoppfadens käme. Eine Binde mit Dehnungsstop schließt die Möglichkeit einer Überdehnung der Binde aus. Bei einer Vorsehung einer Dehnungsschwelle steigt bei Erreichen der Dehnungsschwelle die notwendige Kraft zur weiteren Dehnung schlagartig deutlich an, so dass hierdurch eine Signalwirkung erreicht wird. Die Gefahr einer falschen Anlegung wird bei einer Binde mit Dehnungsschwelle zwar nicht vollständig ausgeschlossen, jedoch deutlich minimiert.

Eine derartige elastische Binde kann insbesondere eine Kompressionsbinde sein, wie sie vorzugsweise im Bereich der Behandlung von venösen und lymphatischen Leiden eingesetzt wird. Derartige Binden sind z. B. Kurzzugbinden, um ein Abschnüren der Blutzirkulation in der Gliedmaße, an der sie angelegt wird, zu vermeiden. Die Erfindung ist gleichermaßen bei Langzugbinden realisierbar, wie sie vorzugsweise zur Behandlung von venösen und lymphatischen Leiden eingesetzt werden.

Dabei soll eine derartige Binde eine elastisch reversible Formveränderung beim Anlegen vollziehen und besitzt vorzugsweise eine Rückstellung von größer 50 %, so dass ein mehrfaches Verwenden ohne Verlust der Kompressionswirkung möglich ist.

Im Folgenden sind Beispiele für entsprechende Gewebe sowie Stoppfäden angegeben, wie sie bevorzugt zur Realisierung einer Kompressionsbinde gemäß der Erfindung eingesetzt werden können:
a) Kompressionsbinde Kurzzugbinde:
   Dehnung bei 10 N/cm, Bindenbreite 90%
   Breite 10 cm,
   gedehnte Länge 5 m,
   Material der Grundbinde: 100% Baumwolle,
   Aufbringung der Stoppfäden, mit Flottierungen, bzw. Längenreserve dergestalt, dass die Fäden bei 50% Dehnung zum Einsatz kommen und dann eine spürbare Krafterhöhung bewirken.
   Material Stoppfaden: Dasselbe hochgedrehte Baumwollzwirngarn wie in dem Grundmaterial.
b) Kompressionsbinde Langzugbinde:
   Dehnung bei 10 N/cm, Bindenbreite 150%
   Breite 10 cm,
   gedehnte Länge 7,5 m.
   Material der Grundbinde: 82% Baumwolle, 13% Polyamid, 5% Elasthan.
   Aufbringung der Stoppfäden, mit Flottierungen, bzw. Längenreserve dergestalt, dass die Fäden bei 50% Dehnung zum Einsatz kommen und dann eine spürbare Krafterhöhung bewirken.
   Material Stopp-/Schwellenfaden: Dasselbe Polyamid-Elasthan-Umwindegarn wie im Grundmaterial.

Die Erfindung soll anhand einer Zeichnung näher erläutert werden. Dabei zeigen:
Figur 1 eine Ausführungsform einer erfindungsgemäßen Binde mit aufgenähtem Stoppfaden;
Figur 2 Kraftdehnungsdiagramme für eine Kurzzug- bzw. Langzugbinde und
Figur 3 ein Kraftdehnungsdiagramm.

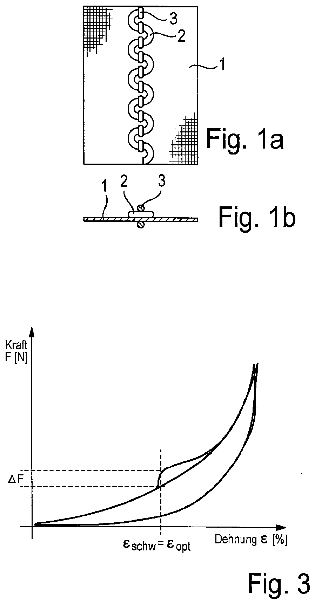

Figur 1 zeigt in einer Darstellung a) einen Ausschnitt aus der erfindungsgemäßen Binde mit einem gewebten Flächengebilde 1 umfassend elastische Kett- sowie vorzugsweise unelastische Schussfäden. Auf dem Flächengebilde ist ein Stoppfaden 2 mittels eines Fixierfadens 3 angebracht. Bei dem Stoppfaden kann es sich vorzugsweise um hochgedrehte Baumwollzwirne oder mit Umwindegarn umwundene Elasthanfäden handeln, wobei das Umwindegarn aus Polyamid oder Baumwolle bestehen kann.

Die Fixierfäden können dabei handelsübliche Stick- oder Nähfäden sein, wie sie in der Bekleidungsindustrie verwendet werden. Diese können z.B. Polyamiden umfassen.

Das Aufbringen des Stoppfadens erfolgt hierbei bei gedehnter elastischer Binde, wobei die elastische Binde bei Aufbringen des Fixierfadens so weit gedehnt wird, dass eine gewünschte Kompressionskraft erreicht wird und diese Dehnung dann als Dehnungsschwelle oder Dehnungsgrenze bei weiteren Verwendungen angezeigt werden soll. Der Stoppfaden wird dann an mehreren Punkten, die insbesondere äquidistant über die Bindenlänge verteilt sind, mit dem Flächengebilde verbunden, wobei der Stoppfaden während des Verbindens annähernd fadengerade in Längsrichtung des Flächengebildes 1 verläuft. Nach Relaxieren des Flächengebildes 1 legt sich der Stoppfaden 2 dann in Wellen bzw. liegt geschlängelt, so dass eine Fadenreserve auf dem Flächengebilde 1 aufliegt. Wird dann das Flächengebilde 1 erneut beim Anlegen der elastischen Binde gestrafft, strafft sich ebenfalls der Stoppfaden bis zur vorgegebenen Dehnungsschwelle bzw. dem vorgegebenen Dehnungsstopp (Dehnungsgrenze).

Alternativ kann der Stoppfaden auch bei der ungedehnten Binde aufgebracht werden. Dies kann z.B. dann vorgesehen sein, wenn der Stoppfaden selber eine Elastizität aufweist und inbesondere die Dehnungsschwell oder -grenze durch die Dehnungsgrenze des Stoppfadens bestimmt ist. Dieser weißt dabei eine Elastizität auf, die geringer als die Elastizität der Binde ist.

Auf diese Weise ist es möglich, zu verhindern oder zumindest die Gefahr zu reduzieren, dass Binden mit zu hoher Dehnung angelegt werden, was zu einer schwerwiegenden gesundheitlichen Störung desjenigen führen kann, an den die Binde angelegt wurde. Darüber hinaus kann jedoch auch vermieden werden, dass die Binde mit einer zu niedrigen notwendigen Dehnung angelegt wird, so dass die Bindenfunktion dann nicht realisiert werden kann.

Die zweite Darstellung b) in Figur 1 zeigt eine entsprechende elastische Binde in einem Querschnitt. Der Stoppfaden 2 ist hierbei mit einer erheblich größeren Fadenstärke und auch hinsichtlich der Farbgestaltung sich abhebend vom Flächengebilde 1 ausgebildet, so dass auch hier einfach für einen Anleger einer entsprechenden Binde optisch zu erkennen ist, ob eine Dehnungsgrenze oder -schwelle erreicht ist.

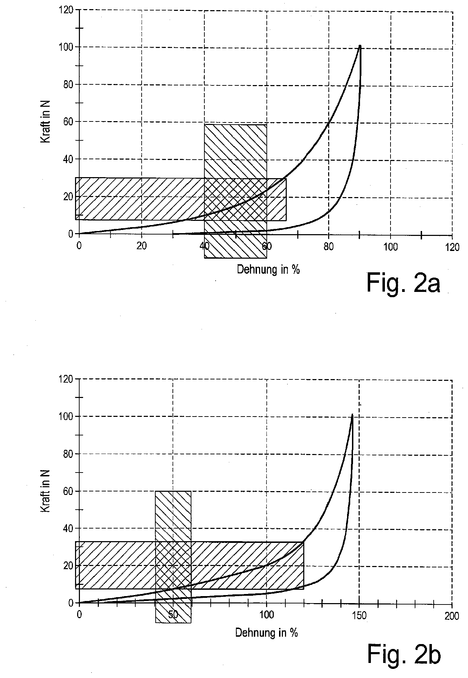

Figur 2 zeigt in den Darstellungen a) und b) Kraftdehnungsdiagramme für Kurzzug- bzw. Langzugbinden. Dabei zeigt Darstellung a) bei einer Kurzzugbinde, wie sie im Beispiel a vorstehend beschrieben ist, das Kraftdehnungsverhalten für die Dehnung sowie die Rückstellung. Dabei ist der gewünschte Dehnbereich sowie der übliche Kraftbereich beim Anlegen markiert dargestellt. Der Kraftbereich verläuft dabei horizontal, der Dehnbereich vertikal. In Darstellung b) wird ein analoges Kraftdehnungsdiagramm für eine Langzugbinde ebenfalls bei einer Belastung von 10 N/cm angegeben, wobei auch hier der gewünschte Dehnbereich sowie der übliche Kraftbereich beim Anlegen markiert sind. Ebenfalls zu erkennen ist ein Bereich, in dem eine Überdehnung möglich ist, so dass deutlich wird, dass insbesondere bei Langzugbinden die Vorsehung einer Dehnschwelle bzw. eines Dehnstopps besonders gut geeignet ist, um Fehlanlegungen zu verhindern. Der Bereich, in dem eine Überdehnung möglich wird, ist der Teil des Kraftbereichs beim Anlegen, der in der Darstellung rechts vom markierten Dehnbereich liegt.

Die Langzugbinde entspricht daher der im Beispiel b vorstehend dargestellten.

Figur 3 zeigt ein Kraftdehnungsdiagramm, wobei mit der durchgezogenen Linie eine Binde mit herkömmlichem Kraftdehnungsverhalten dargestellt ist und über die strichpunktierte Linie eine Binde mit einer Dehnungsschwelle ε_{schw}, wobei ε_{schw} der optimalen gewünschten Dehnung εₒₚₜ entspricht.

Gut zu erkennen ist, dass bei Erreichen der Dehnungsschwelle ε_{schw} die Kraft zur benötigten weiteren Dehnung sprunghaft um den Wert ΔF ansteigt und hierdurch einem Benutzer signalisiert, dass die Dehnungsschwelle erreicht ist.

Weitere Vorteile und Merkmale der Erfindung ergeben sich aus den übrigen Anmeldungsunterlagen.

## Patentansprüche

1. Elastische Binde umfassend ein gewebtes Flächengebilde (1) mit Schussfäden sowie elastischen Kettfäden, wodurch die Binde eine Elastizität in Längsrichtung erhält, **dadurch gekennzeichnet, dass** auf das gewebte Flächengebilde (1) wenigstens ein Stoppfaden (2) aufgebracht ist, durch den eine vorgegebene Dehnungsgrenze oder Dehnungsschwelle der Binde anzeigbar ist, der bei einer vorgegebenen Dehnungsgrenze oder Dehnungsschwelle die weitere Dehnung der elastischen Binde begrenzt oder zumindest erschwert.

2. Elastische Binde nach Anspruch 1, **dadurch gekennzeichnet, dass** der mindestens eine Stoppfaden (2) unelastisch ist oder eine Elastizität aufweist, die geringer ist, als die Elastizität des gewebten Flächengebildes (1).

3. Elastische Binde nach Anspruch 1 oder 2, **dadurch gekennzeichnet, dass** die Schussfäden elastisch und/oder unelastisch sind.

4. Elastische Binde nach einem oder mehreren der vorangehenden Ansprüche, **dadurch gekennzeichnet, dass** die Stoppfäden (2) mit dem gewebten Flächengebilde (1) über Aufnähen, Übernähen, Aufsticken oder Verschweißen oder Verkleben verbunden sind.

5. Elastische Binde nach einem oder mehreren der vorangehenden Ansprüche, **dadurch gekennzeichnet, dass** die Stoppfäden (2) auf die bis zur Dehnungsgrenze oder Dehnungsschwelle vorgedehnte Binde aufbringbar sind in einem gestreckten Zustand des mindestens einen Stoppfadens (2) und die Binde danach relaxierbar ist.

6. Elastische Binde nach einem oder mehreren der vorangehenden Ansprüche, **dadurch gekennzeichnet, dass** über die Anzahl und/oder die Platzierung des mindestens einen Stoppfadens (2) die Dehnungsschwelle und/oder Dehnungsgrenze einstellbar ist.

7. Elastische Binde nach einem oder mehreren der vorangehenden Ansprüche, bei der die Binde in Längsrichtung eine Dehnbarkeit von 20% bis 100%, und insbesondere von 30% bis 90% und insbesondere von 30% bis 70% bei Erreichen der Dehnungsschwelle und/oder Dehnungsgrenze aufweist.

8. Elastische Binde nach einem oder mehreren der vorangehenden Ansprüche, **dadurch gekennzeichnet, dass** die Kettfäden aus elastischen Garnen und/oder Zwirnen bestehen, insbesondere aus hochgedrehten Baumwollzwirnen oder aus Baumwollgarnen mit polyamidumwundenen Elasthanfäden.

9. Elastische Binde nach einem oder mehreren der vorangehenden Ansprüche, **dadurch gekennzeichnet, dass** die Schussfäden aus natürlichen oder synthetischen Garnen, insbesondere aus Baumwollgarnen, Viskosegarnen oder Polyestergarnen bestehen.

10. Elastische Binde nach einem oder mehreren der vorangehenden Ansprüche, **dadurch gekennzeichnet, dass** der mindestens eine Stoppfaden (2) aus hochgedrehten Baumwollzwirnen oder mit Umwindegarn umwundenen Elasthanfäden gebildet ist, wobei das Umwindegarn aus Polyamid oder Baumwolle besteht.

11. Elastische Binde nach einem oder mehreren der vorangehenden Ansprüche, **dadurch gekennzeichnet, dass** der mindestens eine Stoppfaden (2) farblich und/oder über die Fadenstärke von den Kettfäden oder Schussfäden unterscheidbar ist.

12. Elastische Binde nach einem oder mehreren der vorangehenden Ansprüche, **dadurch gekennzeichnet, dass** es sich um eine Kompressionsbinde handelt.

## Claims

1. Elastic bandage, comprising a woven fabric (1) having weft strands and elastic warp strands, which give the bandage elasticity in the longitudinal direction, **characterized in that** at least one stop strand (2) is attached to the woven fabric (1), by means of which strand a predetermined stretch limit or stretch threshold of the bandage can be displayed and which strand limits, or at least impedes, further stretching of the elastic bandage at a predetermined stretch limit or stretch threshold.

2. Elastic bandage according to claim 1, **characterized in that** the at least one stop strand (2) is inelastic or has a lower elasticity than the woven fabric (1).

3. Elastic bandage according to either claim 1 or 2, **characterized in that** the weft strands are elastic and/or inelastic.

4. Elastic bandage according to one or more of the preceding claims, **characterized in that** the stop strands (2) are connected to the woven fabric (1) by being sewn on, by topstitching, by embroidery, or by welding or bonding.

5. Elastic bandage according to one or more of the preceding claims, **characterized in that** the stop strands (2) can be attached to the bandage when it has been pre-stretched to the stretch limit or stretch threshold and when the at least one stop strand (2) is in a stretched state, and the bandage can then be relaxed.

6. Elastic bandage according to one or more of the preceding claims, **characterized in that** the stretch threshold and/or stretch limit can be set via the number and/or the placement of the at least one stop strand (2).

7. Elastic bandage according to one or more of the preceding claims, wherein the bandage stretches in the longitudinal direction by 20% to 100%, in particular 30% to 90%, and in particular 30% to 70% when the stretch threshold and/or stretch limit is reached.

8. Elastic bandage according to one or more of the preceding claims, **characterized in that** the warp strands consist of elastic threads and/or yarns, in particular of high-twisted cotton yarns or of cotton threads having polyamide-wrapped elastane strands.

9. Elastic bandage according to one or more of the preceding claims, **characterized in that** the weft strands consist of natural or synthetic threads, in particular cotton threads, viscose threads, or polyester threads.

10. Elastic bandage according to one or more of the preceding claims, **characterized in that** the at least one stop strand (2) is formed from high-twisted cotton yarns or with elastane strands wrapped with wrapping thread, the wrapping thread being made of polyamide or cotton.

11. Elastic bandage according to one or more of the preceding claims, **characterized in that** the at least one stop strand (2) can be distinguished from the warp strands or weft strands by color and/or strand thickness.

12. Elastic bandage according to one or more of the preceding claims, **characterized in that** it is a compression bandage.

## Revendications

1. Bande élastique comprenant une surface tissée (1) avec des fils de trame ainsi que des fils de chaîne élastiques, ce qui a pour effet que la bande reçoit une élasticité dans la direction longitudinale, **caractérisée en ce qu'**au moins un fil d'arrêt (2) est appliqué sur la surface tissée (1), par lequel une limite d'extension ou un seuil d'extension prédéfini(e) de la bande peut être indiqué(e), qui pour une limite d'extension ou un seuil d'extension prédéfini(e) limite ou au moins complique l'extension supplémentaire de la bande élastique.

2. Bande élastique selon la revendication 1, **caractérisée en ce que** le au moins un fil d'arrêt (2) est non élastique ou présente une élasticité qui est inférieure à l'élasticité de la surface tissée (1).

3. Bande élastique selon la revendication 1 ou 2, **caractérisée en ce que** les fils de trame sont élastiques et/ou non élastiques.

4. Bande élastique selon l'une ou plusieurs des revendications précédentes, **caractérisée en ce que** les fils d'arrêt (2) sont reliés à la surface tissée (1) par couture, surpiqure, broderie ou soudure ou collage.

5. Bande élastique selon l'une ou plusieurs des revendications précédentes, **caractérisée en ce que** les fils d'arrêt (2) peuvent être appliqués sur la bande prétendue jusqu'à la limite d'extension ou au seuil d'extension dans un état étiré du au moins un fil d'arrêt (2) et la bande peut ensuite être relâchée.

6. Bande élastique selon l'une ou plusieurs des revendications précédentes, **caractérisée en ce que** le seuil d'extension et/ou la limite d'extension sont réglables par l'intermédiaire du nombre et/ou du placement du au moins un fil d'arrêt (2).

7. Bande élastique selon l'une ou plusieurs des revendications précédentes, pour laquelle la bande présente dans la direction longitudinale une extensibilité de 20 % à 100 %, et en particulier de 30 % à 90 % et en particulier de 30 % à 70 % lors de l'atteinte du seuil d'extension et/ou de la limite d'extension.

8. Bande élastique selon l'une ou plusieurs des revendications précédentes, **caractérisée en ce que** les fils de chaîne sont constitués de fils élastiques et/ou fils retors, en particulier de fils retors de coton hautement tordus ou de fils de coton avec des fils d'élasthanne guipés de polyamide.

9. Bande élastique selon l'une ou plusieurs des revendications précédentes, **caractérisée en ce que** les fils de trame sont constitués de fils naturels ou synthétiques, en particulier de fils de coton, de fils de viscose ou fils de polyester.

10. Bande élastique selon l'une ou plusieurs des revendications précédentes, **caractérisée en ce que** le au moins un fil d'arrêt (2) est formé de fils retors de coton hautement tordus ou de fils d'élasthanne guipés avec un fil de guipage, dans laquelle le fil de guipage est constitué de polyamide ou de coton.

11. Bande élastique selon l'une ou plusieurs des revendications précédentes, **caractérisée en ce que** le au moins un fil d'arrêt (2) peut se distinguer des fils de chaîne ou fils de trame par la couleur et/ou par l'épaisseur du fil.

12. Bande élastique selon l'une ou plusieurs des revendications précédentes, **caractérisée en ce qu'**il s'agit d'une bande de compression.
